# EUROPEAN PATENT SPECIFICATION

(11) **EP 0 507 513 B1**
(45) Date of publication and mention of the grant of the patent: **27.11.1996**
(21) Application number: 92302691.8
(22) Date of filing: 27.03.1992
(51) Int. Cl.: A61F 5/02

(54) **Therapeutic device**
Therapeutische Vorrichtung
Dispositif thérapeutique

(30) Priority: 03.04.1991 US 678938; 03.07.1991 US 727221
(43) Date of publication of application: 07.10.1992
(73) Proprietor: Fisher Scientific Company, Pittsburgh, PA 15219 (US)
(72) Inventor: Sebastian, Peter R., Salisbury, Maryland 21801 (US); Sebastian, Thomas V., Reading, Pennsylvania 19611 (US)
(74) Representative: Perkins, Sarah

(56) References cited:
- EP-A- 0 261 809
- FR-A- 2 114 033
- US-A- 3 441 027
- US-A- 4 022 197
- US-A- 4 475 543

## Description

The present invention relates to a therapeutic appliance for application to the lumbar area and upper portion of the sacrum as specified in the preamble of Claim 1. Such an appliance is e.g. known from US-A-3441027 or EP-A-0261809.

Low back pain commonly arises in individuals due to alterations in the biomechanics of the lumbosacral spine and its supporting muscular and ligamentous structures. Therapeutic appliances can be useful, both in the treatment and prevention of back pain in individuals, when the primary mechanism involved in the generation of pain is due to muscular strain, spasm, or fatigue. The resulting pain can be produced within the muscles themselves or from the secondary effects from loss of adequate and balanced support to the osseous and ligamentous structures of the lower back, whose movements they regulate.

Known supports have been designed in an attempt to limit fatigue and undue strain in the above-mentioned structures and to aid in the restoration of both normal and balance muscle tone. While such aims are rarely achieved by the application of known therapeutic appliances alone, such appliances are often helpful in the prevention and treatment of various lower back conditions.

However, even these appliances are apt to exhibit one or more significant disadvantages. For example some supports contain rigid elements or narrow elastic bands which will often dig into the skin or produce irritation over bony protuberances. This will often lead to noncompliance by the user. Some of the currently available therapeutic appliances designed for application to the lower back fail to provide support over the entire lumbar area, either having too narrow a width or inadequate supporting elements at the top and/or bottom of the device. Other devices are limited in their usefulness by applying support only over the most central areas of the spine.

Further, while rigid immobilization is desirable in certain types of spine injury, excessive limitation of spine movement for the muscular conditions previously described can be unwanted and create weakness through disuse of the supporting musculature.

Further, in known lumbar support appliances utilizing shoulder suspenders, the suspender straps are sewn to the body of the appliance. Such appliances suffer a drawback in that the suspenders can potentially be snagged by a stationary or non-stationary piece of industrial equipment, thus entangling the user with the equipment. Such entanglement could result in serious injury to the user.

In EP-A-0261809 a therapeutic lumbosacral appliance is described which is in the form of a shell having a length sufficient to extend around the abdominal region of the body and having fasteners to secure the ends. The shell includes fixed pockets into which hot or cold packs may be inserted.

An object of the present invention is to provide a therapeutic appliance which is comfortable to wear, which adequately covers the lumbar region and which can provide optimum support and/or therapeutic treatment for the lower back.

To achieve this object, the therapeutic appliance of the present invention comprises a therapeutic appliance securable in an operative orientation around the abdominal region of the body for use in therapeutically treating the lumbar area and upper portion of the sacrum, said appliance comprising:
an external shell having opposite ends, a length as taken in a longitudinal direction of the appliance between said ends that is sufficient to enable said shell to extend around and encircle the abdominal region of the body, a central region between said ends having a width as taken transversely to said longitudinal direction that is sufficient to enable the central region to cover the lumbar area and upper portion of the sacrum, and closure means at said opposite ends for detachably securing the ends together so as to allow the appliance to be detachably secured around the abdominal region of the body; and
pouches attached to said external shell, each pouch having several sides and being located at the central region of said shell to a respective side of the midpoint between said opposite ends of the shell, characterised in that each said pouch is attached to said external shell through only one of said sides of the pouch such that each said pouch is pivotable relative to said external shell about the one side through which the pouch is attached to the shell; each said pouch being pivotable between a first position at which the pouch is disposed at such a location between said midpoint and a respective one of the ends of said shell as to overlie the lumbodorsal fascia and quadratus lumborum muscles when the shell is secured in the operative orientation of the appliance around the abdominal region of the body and a second position at which the pouch is disposed at such a location closer to said midpoint from said first position as to overlie the erector spinae muscles when the shell is secured in the operative orientation of the appliance around the abdominal region of the body.

In addition, a band-like elastic member may be secured to the shell in a state of tension over the pouches whereby the pads accommodated in the pouches will be urged into engagement with the lower back. Opposite ends of the elastic member can be secured at various locations along opposite ends of the external shell whereby the tension of the elastic member can be varied to adjust the tension of the appliance and/or the force at which the pads are urged into engagement with the lower back.

In addition, break away shoulder suspenders may be provided. The shoulder suspenders may be formed of an elastic woven material and secured to the external shell of the therapeutic appliance by hook and loop closures. By the provision of the hook and loop closures, the suspender will tend to break away from the external shell of the therapeutic appliance in the event that the shoulder suspenders are somehow snagged by a piece of equipment. Further, the provision of shoulder suspenders permits the user of the appliance to loosen the fastener of the appliance to allow evaporation of moisture and dissipation of heat. Such shoulder suspenders may be completely detachable from the therapeutic appliance if desired.

The invention will now be described in more detail with reference by way of example to the accompanying drawings, in which:
Figure 1 is an exploded view of an embodiment of a therapeutic appliance according to the present invention;
Figure 2 is a perspective view of the therapeutic appliance shown in Figure 1 but with the pouches shown in a different position and various elements omitted for the sake of clarity;
Figure 3 is a perspective view of the shell of the appliance of Figure 1 as seen from the other side thereof;
Figures 4(a) and (b) are schematic diagrams showing the therapeutic appliance of Figure 1 secured around the abdominal region of the body with the pads assuming positions corresponding to those illustrated in Figure 2 and Figure 1, respectively; and
Figure 5 is a perspective view of the appliance of the present invention including break-away suspenders.

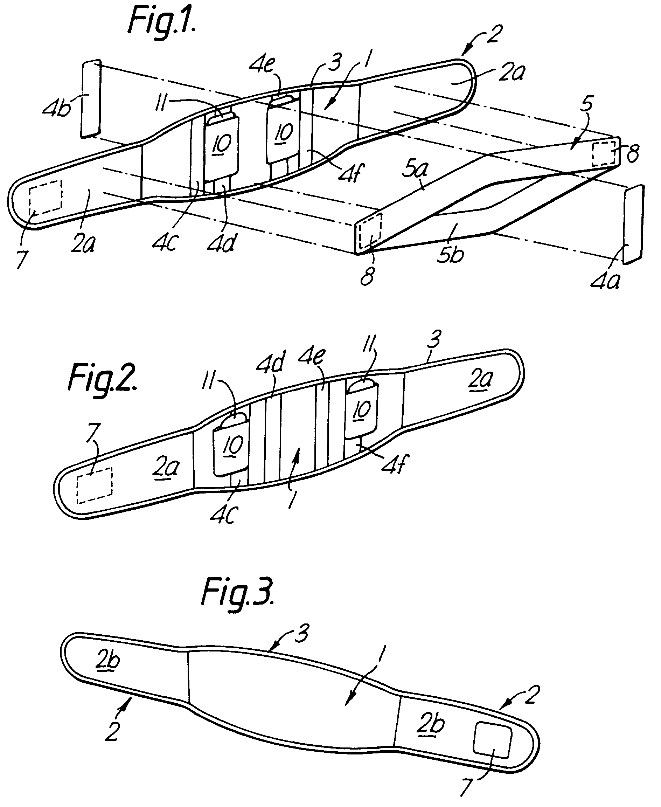

The illustrated therapeutic appliance has an external shell including a central region generally designated by reference numeral 1 and opposite ends designated by reference numeral 2. The external shell, including the central region 1 and opposite ends 2, is fabricated from material having a degree of elasticity and the shape best shown in Figure 3. Such material is preferably a known knit material. At the outer side of the shell shown in Figure 1, such knit material is covered at the opposite ends 2 with pieces of material 2a to be described in more detail below. On the opposite side of the shell, as shown in Figure 3, the ends of the knit material are covered by pieces of plush foam 2b. The pieces of material 2a, the pieces of plush foam 2b and the ends of the knit material interposed therebetween are sewn together so as to constitute the external shell. This may be accomplished by stitching a bias binding 3 at the periphery of the external shell.

A plurality of stiffening members 4a - 4f are also secured to the external shell. The stiffening members 4a - 4f preferably include a semi-rigid strip of KYDEC* material enclosed within a CAMBRELLE* sleeve. The sleeves of the stiffening members 4a - 4f can be secured to the external shell by means of stitching. In this respect, the bias binding 3 is also used to stitch the ends of the sleeves of the stiffening members 4a - 4f to the external shell.
* (trade marks)

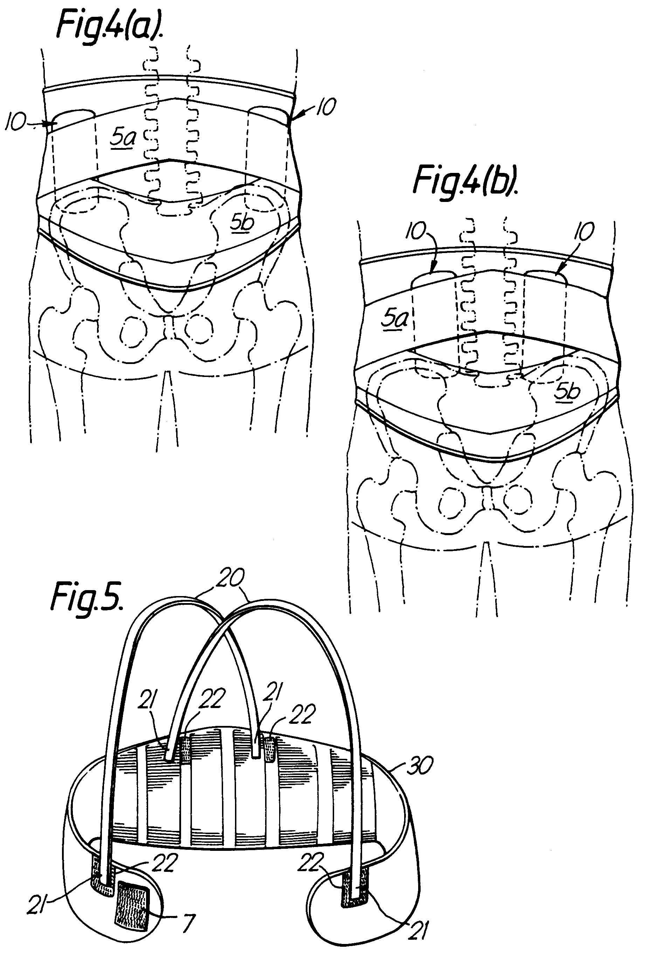

As best seen in Figures 1 and 2, pouches 10, which can be fabricated of CAMBRELLE*, are pivotably attached to the external shell at one side thereof, preferably the outer side facing away from the user of the appliance. Each of the pouches 10 is located at the central region 1 of the shell to a respective side of the midpoint between the opposite ends 2. The pouches 10 are attached at only one side thereof to the belt (the sides adjacent stiffening members 4c, 4f, respectively in Figure 1). Thus, each pouch can be pivotable about one side thereof between a first position shown in Figure 2 in which the pouch is disposed at a location between the longitudinal midpoint of the shell and one end of the shell and a second position shown in Figure 1 at which the pouch is disposed at a location closer to the midpoint than the first position. Turning to Figures 4(a) and 4(b), the first position as shown in Figure 4(a) is one at which the pouch 10 will overlie the lumbodorsal fascia and quadratus lumborum muscles when the shell is secured around the abdominal region of the body. As shown in Figure 4(b) the second position is one at which the pouches will overlie the erector spinae muscles when the shell is secured around the abdominal region of the body.
* (trade mark)

As is also apparent from Figure 4(a), the central region 1 of the external shell has a width that is sufficient to enable the appliance to cover the lumbar area and the upper portion of the sacrum. And, the lower edge of the appliance is contoured to limit bony bridging from the iliac crest.

The pouches 10 may take the form of a sleeve open at the top thereof so as to accommodate a therapeutic pad 11 of a type necessary to impart a desired therapeutic treatment to the lower back. For instance, the therapeutic pad 11 may be a foam pad for providing support to the lower back, an inflatable air bladder for providing support to the lower back, a heat-retaining pad known per se for radiating therapeutic amounts of heat to the lower back, or a known type of cold-pack for applying therapeutic amounts of cold to the lower back. Thus, the therapeutic appliance of the present invention may be provided as a kit including a plurality of pads of any of the above-mentioned types which may be suitably selected and inserted into the pouches 10 to carry out a desired therapeutic treatment.

It should thus be quite apparent that because the pouches can be positioned medially over the erector spinae muscles or laterally over the lumbodorsal fascia and quadratus lumborum muscles, the therapeutic pads 11 accommodated in the pouches 10 can likewise be positioned medially over the erector spinae muscles or laterally over the lumbodorsal fascia and quadratus lumborum muscles. In addition, and again depending upon the particular case at hand, the pouches 10 can be filled with a therapeutic pad 11 on one or both sides of the appliances or can even remain empty.

Referring once again to Figure 1, a band-like elastic member 5 is attached at a central portion thereof to the external shell between the stiffening members 4a, 4b. Although the stiffening members 4a, 4b are shown as being attached to opposite sides of the appliance with the central portion of the elastic member 5 interposed therebetween, in practice, the stiffening member 4b may be disposed on the same side of the belt as the stiffening member 4a but with the central portion of the elastic member 5 still interposed between members 4a, 4b.

In the embodiment shown in the figures, the band-like elastic member 5 is in the form of a loop of knitted elastic material having upper and lower elastic band sections 5a, 5b which will be described in more detail below. The external shell and the elastic member include fasteners at the ends thereof for detachably securing the ends of the elastic member 5 to various locations along the ends 2 of the external shell. For example, the fasteners can be well-known hook and loop types of fasteners, such as VELCRO* fasteners, by forming the pieces of material 2a as loop-type fastener members and by providing the ends of the band-like elastic member 5 with hook-type fastener members 8.
* (trade mark)

With the ends of the band-like elastic member 5 secured to the ends 2 of the external shell, the elastic member 5 extends over the central region 1 of the external shell in a state of tension over the pouches 10 whereby any pads 11 accommodated in the pouches 10 will be urged into engagement with the lower back. Because the opposite ends of the elastic member 5 can be secured to various locations along opposite ends 2 of the external shell, the tension of the elastic member 5 can be varied to adjust the force at which the pads 11 are urged into engagement with the lower back or simply to adjust the tension of the appliance around the abdominal region.

Referring once again to Figures 4(a) and 4(b), the upper elastic band 5a extends across the central region 1 of the external shell at such a location as to be aligned transversely across the lumbar area when the appliance is secured around the abdominal region. The lower elastic band 5b is inclined upwardly from the central portion of member 5 toward the upwardly elastic band 5a.

In addition, it should be noted that closure members are provided at the opposite ends 2 of the external shell for detachably securing the ends together so as to allow the appliance to be detachably secured around the abdominal region of the body in the position schematically illustrated in Figures 4(a) and 4(b). If the pieces of material 2a are in the form of loop-type fastening members as described above, a hook-type fastening member 7 may be secured at one end of the shell at the side thereof opposite to the side at which the loop-type fastening members are exposed. In this way, the loop-type fastening members 2a, constituting a means for fastening the ends of the elastic member 5 to the ends of the external shell, will also constitute closure means with the hook-type fastening member 7 for allowing the appliance to be detachably secured around the abdominal region of the body.

From the above-described description of the preferred embodiment, it should be clear that the present invention exhibits the following features and advantages, among others:
(1) the central region of the appliance has a width which is sufficient to cover the lumbar area and upper portion of the sacrum;
(2) the lower edge of the shell is contoured to limit bony bridging from the iliac crest;
(3) an upper tension-exerting elastic band is aligned transversely across the lumbar area while the lower elastic band is angled upwardly and laterally;
(4) pouches are provided on both sides of the midpoint to accommodate removable adjustable-pressure air bladders, foam pads, and/or therapeutic thermal pouches utilizing hot or cold;
(5) the pouches can be positioned medially over the erector spinae muscles or laterally over the lumbodorsal fascia and quadratus lumborum muscles; and
(6) the pouches can be filled with a therapeutic pad on one or both sides or remain empty.

For general support or injury prevention purposes, it is advisable to fill and position the pouches in a similar manner to maintain a balanced support over the lower back musculature and spinal structures. However, in certain therapeutic situations where an unbalance in muscular support or spasm exists, the type of therapeutic pad and/or the symmetry of the pouches can be varied between the two sides until the desired therapeutic goal is achieved. Further, adjustment of the support on either or both sides can be achieved by repositioning either of the ends of the elastic member on the ends of the external shell to adjust the tension exerted by the elastic member.

Finally, it should be noted that various other changes and modifications will become apparent to those of ordinary skill in the art reviewing the detailed description above. For instance, the pouches 10 and central region 1 of the external shell may be provided with cooperating fasteners to detachably secure the pouches in the desired position thereof.

Referring now to Figure 5, the break away shoulder suspenders of the present invention will be described.

In the preferred embodiment, suspender straps 20 are provided of an elastic woven material. The width and the length of the straps 20 may vary depending on the size of the user and/or the amount of weight which the suspenders are to support. Hook-type fastening members 21 are provided at each end of the straps 20. In the preferred embodiment, the hook-type fastening members 21 face inwardly toward the lumbar support appliance 30 when worn. Affixed to the lumbar support appliance 30 are loop-type fasteners 22. The loop-type fasteners face outwardly from the lumbar support appliance 30 to engage the hook-type fasteners 21 of the straps 20.

The connecting area of each of the loop-type fastening members 22 may be provided in such a size as to permit height adjustment and side-to-side adjustment of the straps 20. For example, the width of each loop type fastening member 22 can be wider than that of each respective hook-type fastener 21. Consequently, the shoulder suspenders can be positioned to maximize comfort and to minimize potential entanglements.

Optionally, the straps of the suspenders may be affixed to each other at a location corresponding to the back and below the shoulder of the user where the straps 20 cross over as shown in Figure 5.

By the provision of the break away shoulder suspenders of the present invention, it should be clear that the following features and advantages, among others, are obtained:
(1) potential injury to the user resulting from snagging of the suspenders is reduced by the provision of the hook-type and loop-type fastening members;
(2) the loop-type fastening members are provided in such a manner as to permit vertical and side-to-side adjustments of the shoulder suspenders to account for varying sizes of upper torsos of individual users, to maximize overall comfort and to minimize potential entanglements;
(3) the lumbar support device can be loosened to permit evaporation of moisture and dissipation of heat while conveniently allowing the user to position and/or store the support device on the user; and
(4) in the event that the shoulder suspenders are not desired, the shoulder suspenders can be simply detached from the body of the lumbar support device.

## Claims

1. A therapeutic appliance securable in an operative orientation around the abdominal region of the human body for use in therapeutically treating the lumbar area and upper portion of the sacrum, said appliance comprising:
an external shell having opposite ends (2), a length as taken in a longitudinal direction of the appliance between said ends that is sufficient to enable said shell to extend around and encircle the abdominal region of the body, a central region (1) between said ends (2) having a width as taken transversely to said longitudinal direction that is sufficient to enable the central region to cover the lumbar area and upper portion of the sacrum, and closure means (2a,7) at said opposite ends for detachably securing the ends together so as to allow the appliance to be detachably secured around the abdominal region of the body; and
pouches (10) attached to said external shell, each pouch having several sides and being located at the central region (1) of said shell to a respective side of the midpoint between said opposite ends (2) of the shell, characterised in that each said pouch is attached to said external shell through only one of said sides of the pouch such that each said pouch is pivotable relative to said external shell about the one side through which the pouch is attached to the shell; each said pouch being pivotable between a first position at which the pouch is disposed at such a location between said midpoint and a respective one of the ends of said shell as to overlie the lumbodorsal fascia and quadratus lumborum muscles when the shell is secured in the operative orientation of the appliance around the abdominal region of the body and a second position at which the pouch is disposed at such a location closer to said midpoint from said first position as to overlie the erector spinae muscles when the shell is secured in the operative orientation of the appliance around the abdominal region of the body.

2. A therapeutic appliance as claimed in claim 1, and further comprising a band-like elastic member (5) extending over the central region (1) of said shell and having opposite ends secured to opposite ends (2) of said shell.

3. A therapeutic appliance as claimed in claim 2, wherein said shell and said elastic member (5) include fastening means (8) at the ends thereof for allowing the ends of said elastic member to be detachably secured to said shell at various locations along the ends of said shell.

4. A therapeutic appliance as claimed in claim 2, wherein said elastic member includes an upper elastic band (5a) extending across said central region (1) at such a location as to be aligned transversely across the lumbar area when the appliance is secured in the operative orientation around the abdominal region, and a lower elastic band (5b) inclined upwardly from a central section thereof toward said upper elastic band (5a).

5. A therapeutic appliance as claimed in claim 1, and further comprising a kit including a plurality of pads (11) selected from the group consisting of foam pads, inflatable air bladders, heat retaining means for radiating heat, and cold-pack means for applying cold, each of the pads of said kit insertable into the pouches attached to said shell.

6. A therapeutic appliance as claimed in claim 1, further comprising:
first fastener members secured to said external shell; and
suspender straps each having opposite first and second ends, each of the opposite first and second ends having a second fastener member for releasably engaging the respective first fastener members secured to the external shell;
wherein said first fastener members are one of hook-type and loop-type fasteners and said second fastener members are the other of hook-type and loop-type fasteners.

## Patentansprüche

1. Eine therapeutische Vorrichtung, die in einer funktionellen Orientierung um die Bauchregion des menschlichen Körpers für den Gebrauch bei der therapeutischen Behandlung der Lendengegend und der oberen Partie des Sacrums befestigt werden kann, besagte Vorrichtung umfaßt:
eine externe Hülle, die entgegengesetzte Enden (2) aufweist, eine Länge wie in Längsrichtung der Vorrichtung zwischen den besagten Enden gemessen, die ausreichend ist, um der besagten Hülle zu gestatten sich um die Bauchregion des Körpers zu erstrecken und diese zu umschließen, eine zentrale Region (1)zwischen den besagten Enden (2), die eine Breite aufweist wie quer zu der besagten Längsrichtung gemessen, die ausreichend ist, um der zentralen Region zu ermöglichen die Lendengegend und die obere Partie des Sacrums abzudecken, und Verschlußmittel (2a,7) an besagten entgegengesetzten Enden zum lösbaren Zusammenschluß der Enden, um zu gestatten, daß die Vorrichtung lösbar um die Bauchregion des Körpers befestigt werden kann; und
Taschen (10), die an der besagten externen Hülle angebracht sind, jede Tasche weist mehrere Seiten auf und ist in der zentralen Region (1) der besagten Hülle an einer entsprechenden Seite des Mittelpunkts zwischen besagten entgegengesetzten Enden (2) der Hülle befestigt, dadurch gekennzeichnet, daß jede besagte Tasche an der besagten externen Hülle nur durch eine der besagten Seiten der Tasche dermaßen befestigt ist, daß jede besagte Tasche relativ zur besagten externen Hülle um die eine Seite schwenkbar ist durch welche die Tasche an der Hülle befestigt ist; jede besagte Tasche ist zwischen einer ersten Position, in der die Tasche an solch einer Stelle zwischen dem besagten Mittelpunkt und einem entsprechenden der Enden der besagten Hülle angeordnet ist, um die lumbodorsalen Faszien- und Quadratus-Lumborummuskeln zu überlagern, wenn die Hülle in der funktionellen Orientierung der Vorrichtung um die Bauchregion des Körpers befestigt ist, und einer zweiten Position schwenkbar ist, in der die Tasche an solch einer Stelle angeordnet ist, die dem besagten Mittelpunkt ab der besagten ersten Position näher liegt, um die Aufrichtmuskeln der Wirbelsäule zu überlagern, wenn die Hülle in der funktionellen Orientierung der Vorrichtung um die Bauchregion des Körpers befestigt ist.

2. Eine therapeutische Vorrichtung wie in Anspruch 1 beansprucht, und außerdem ein bandartiges elastisches Element (5) umfassend, das sich über die zentrale Region (1) der besagten Hülle erstreckt und entgegengesetzte Enden aufweist, die an entgegensetzten Enden (2) der besagten Hülle befestigt sind.

3. Eine therapeutische Vorrichtung wie in Anspruch 2 beansprucht, worin die besagte Hülle und das besagte elastische Element (5) Befestigungsmittel (8) an dessen Enden einschließen, um den Enden des besagten elastischen Elements zu gestatten an der besagten Hülle an verschiedenen Stellen entlang den Enden der besagten Hülle lösbar befestigt zu werden.

4. Eine therapeutische Vorrichtung wie in Anspruch 2 beansprucht, worin das besagte elastische Element ein oberes elastisches Band (5a) einschließt, das sich quer über die besagte zentrale Region (1) an solch einer Stelle erstreckt, daß es quer über die Lendengegend ausgerichtet ist, wenn die Vorrichtung in der funktionellen Orientierung um die Bauchregion befestigt ist, und ein unteres elastisches Band (5b), daß ab einem zentralen Abschnitt davon in Richtung des oberen elastischen Bandes (5a) schräg nach oben verläuft.

5. Eine therapeutische Vorrichtung wie in Anspruch 1 beansprucht, und außerdem eine Ausrüstung einschließlich einer Vielheit von Polstern (11) umfassend, die aus einer Gruppe ausgewählt wurden, die aus Schaumstoffpolstern, aufpumpbaren Luftbälgen, Wärme zurückhaltenden Mitteln für die Ausstrahlung von Wärme, und Kältepacks für die Aufbringung von Kälte besteht, wobei sich jedes der Polster der besagten Ausrüstung in die an der besagten Hülle angebrachten Taschen einschieben läßt.

6. Eine therapeutische Vorrichtung wie in Anspruch 1 beansprucht, die außerdem umfaßt:
erste Befestigungselemente, die an der besagten externen Hülle angebracht sind; und
Trägerbänder, jedes mit entgegengesetzten ersten und zweiten Enden, jedes der entgegengesetzten ersten und zweiten Enden weist ein zweites Befestigungselement für lösbaren Eingriff mit den entsprechenden ersten Befestigungselementen auf, die an der externen Hülle befestigt sind;
worin besagte erste Befestigungselemente eines Typs von hakenartigen und schlingenartigen Verschlüssen und besagte zweite Befestigungselemente des anderen Typs von hakenartigen und schlingenartigen Verschlüssen sind.

## Revendications

1. Un dispositif thérapeutique que l'on peut attacher selon une orientation de travail autour de la région abdominale du corps humain, afin d'appliquer un traitement thérapeutique à la région lombaire et à la partie supérieure du sacrum, ledit dispositif comprenant:
une coquille externe comportant des extrémités opposées (2), possédant une longueur qui, mesurée dans la direction longitudinale du dispositif entre lesdites extrémités, soit suffisante pour permettre à ladite coquille de se prolonger autour de la région abdominale du corps et d'entourer cette dernière, une partie centrale (1) située entre lesdites extrémités (2) et d'une largeur qui, lorsqu'elle est mesurée de manière transversale par rapport à ladite direction longitudinale, soit suffisante pour permettre à la partie centrale de recouvrir la région lombaire et la partie supérieure du sacrum, et un dispositif de fermeture (2a,7) situé auxdites extrémités opposées et servant à attacher, ou à détacher, les extrémités afin de fixer, ou de détacher, le dispositif autour de la région abdominale du corps;et
des poches (10) montées sur ladite coquille externe, chaque poche comportant plusieurs côtés et étant située au niveau de la partie centrale (1) de ladite coquille le long d'un côté respectif du point central entre lesdites extrémités opposées (2) de la coquille, caractérisé par le fait que chacune desdites poches est fixée à ladite coquille externe par le biais d'un seul desdits côtés de ladite poche, en sorte que chacune desdites poches puisse pivoter par rapport à ladite coquille externe et autour du côté par lequel la poche est fixée à la coquille; chacune desdites poches pouvant pivoter pour passer d'une première position, selon laquelle la poche est disposée à un endroit entre ledit point central et l'une desdites extrémités de ladite coquille, en sorte que la poche recouvre les faisceaux lombodorsaux et les quadratus lombaires lorsque l'on attache la coquille selon l'orientation de travail du dispositif autour de la région abdominale du corps et une deuxième position, selon laquelle la poche est disposée à un endroit plus proche dudit point central par rapport à la première position, en sorte qu'elle recouvre les muscles erector spinae lorsque l'on attache la coquille selon l'orientation de travail du dispositif autour de la région abdominale du corps.

2. Un dispositif thérapeutique ainsi revendiqué à la revendication 1, comprenant en outre un élément élastique en forme de bande (5) se prolongeant au-delà de la partie centrale (1) de ladite coquille et comportant des extrémités opposées attachées aux extrémités opposées (2) de ladite coquille.

3. Un dispositif thérapeutique ainsi revendiqué à la revendication 2, dans lequel ladite coquille et ledit élément élastique (5) comportent un dispositif de fermeture (8) aux extrémités de l'élastique, afin de pouvoir attacher, ou détacher, les extrémités dudit élément élastique à ladite coquille en plusieurs endroits le long des extrémités de ladite coquille.

4. Un dispositif thérapeutique ainsi revendiqué à la revendication 2, dans lequel ledit élément élastique comporte une bande élastique supérieure (5a) se prolongeant au-dessus de la partie centrale (1) dans une position telle qu'elle soit située dans l'alignement transversal de la région lombaire lorsque l'on attache le dispositif selon son orientation de travail autour de la région abdominale du corps, et une bande élastique inférieure (5b) dirigée vers le haut depuis son milieu et en direction de ladite bande élastique supérieure (5a).

5. Un dispositif thérapeutique ainsi revendiqué à la revendication 1, comprenant en outre un kit comportant un ensemble de coussinets (11) choisis parmi la gamme comprenant les coussinets de mousse, les vessies gonflables, les dispositifs d'émission de chaleur, et les dispositifs de diffusion du froid, chacun des coussinets dudit kit pouvant être introduit dans les poches montées sur ladite coquille.

6. Un dispositif thérapeutique ainsi revendiqué à la revendication 1, comprenant en outre:
des premiers éléments de fermeture fixés sur ladite coquille externe;et
des bretelles de suspension comportant chacune une première et une deuxième extrémités opposées, chacune des première et deuxième extrémités opposées comportant un deuxième élément de fermeture destiné à pouvoir s'enclencher dans les premiers éléments de fermeture respectifs fixés sur la coquille externe, ou à détacher les bretelles;
dans lequel lesdits premiers éléments de fermeture sont un crochet et une boucle et lesdits deuxièmes éléments de fermeture sont un crochet et une boucle complémentaires avec ceux desdits premiers éléments.
